# EUROPEAN PATENT APPLICATION

(11) **EP 3 476 928 A1**
(43) Date of publication of application: **01.05.2019**
(21) Application number: 17020509.0
(22) Date of filing: 27.10.2017
(51) Int. Cl.: C12M 1/34, C12M 1/36, C12M 1/00, D21H 27/00, C12Q 1/04

(54) **DETECTION OF BACTERIAL GROWTH**

(71) Applicant: Envall Consulting AB, 426 79 Västra Frölunda (SE)
(72) Inventor: Håkan, Envall, 426 79 Västra Frölunda (SE)
(74) Representative: Nobeli Business Support AB

(57) **Abstract**

The invention relates to a method (400), a control unit and a system for detection of bacterial growth in a sample of a) pulp or b) cellulose-based board. The method (400) comprises placing (402) the sample of pulp or cellulose-based board in the chamber (6); capturing (403) a first image of the pulp or cellulose-based board sample; capturing (405) a final image of the sample of pulp or cellulose-based board, when a predetermined time period has elapsed since the first image was captured (403); comparing (406) the captured (405) final image with the captured (403) first image; and determining (407) a rate of bacterial growth, based on the made comparison (406).

## Description

The present invention relates to a method and a system for detection of bacterial growth in pulp, in particular in cellulose-based board.

### Background of the invention

When producing different grades of paper and cardboard, characteristics such as cleanliness and quality are of great importance since paper and cardboard often are used as packaging materials for food, beverages etc. and as such, they are often in direct contact with food contained in the packages made of said paper and cardboard. The environment in a paper mill is favorable for bacterial growth. The process conditions at the mill include temperatures from 30º to 55ºC (even warmer systems are available) and a pH between 4-10 resulting in a suitable environment for a large range of bacteria types. Moreover, there is a constant level of nutrients from the pulp (the starting material for paper and board manufacturing) and the various stock chemicals which further may stimulate growth. The bacterial flora in a paper mill is dominated by aerobic species and is very mixed. Bacterial concentration in the pulp may be significantly higher than in the produced board. Bacteria from raw materials and the environment contaminate also the manufacturing machines. These bacteria can cause problems both in the manufacturing process and in the finished paper or board product and they may destroy the components therein. Also, biofilms may negatively affect taste, smell and produce spots in the finished product. There is even a risk that bacteria in the finished paper or board starts to grow and thus contaminate goods/food contained in the packaging materials made of said board thus affecting the smell and taste of said goods/food. It is therefore important to keep the microbial levels at as low levels as possible during manufacture of paper and cardboard. Problems with bacteria in board mainly arises when the bacteria are in sporulated form, because the spores have a significantly higher resistance to high temperatures and survive the passage through the drying section of the production line in the paper mill, a section where vegetative bacteria normally die. Hence, it is of great importance to analyze grown bacterial content of finished board grades so as to control that the content is within an acceptable range. Quality controls are performed throughout the manufacturing line of the board as well as in finished board in order to control the levels of bacteria and spore content. At the time being there are no laws regulating acceptable levels/amounts of bacteria in finished board but only recommendations as well as customer requirements.

Conventionally, testing of food and packaging cardboard for grown bacterial content have been conducted by a manual test based on a visual counting process.
There are specified colony concentrations that must not be exceeded for each certified quality. The conventional ocular testing method results in ergonomic problems for the persons performing the methods and may be time-consuming and not always sufficiently accurate. It would therefore be desirable to provide a process in which a sample of pulp or formed cellulose-based board is withdrawn from the board production process for analysis thereof in a more accurate and time-efficient manner. It would further be desirable to provide such a method which at a predetermined point in time at a suitable stage in a process, e.g. after formation of a board, withdraw a sample on-line while the production process is in operation. It would also be desirable to provide a control unit and a system for performing such a method.

### The invention

It is an object of the present invention to overcome the drawbacks of the prior art. This can be obtained by a method, a control unit and a system as defined in the independent claims. Further preferred embodiments are defined inter alia in the dependent claims.

According to one embodiment, at least one sample is withdrawn from the board manufacturing process at a suitable stage in the process, for example after formation of a board, from the head box or from the stock solution, preferably from a cellulose-based board.

Preferably, the sample, e.g. board sample is worked-up to improve the analysis of the sample, e.g. by removing disturbing substances such as fibres, pigments, added paper chemicals etc. impairing the correctness of the analysis and thus the conditions for performing an accurate determination of the bacterial growth.

According to one embodiment, a predetermined number of samples are withdrawn from the process, e.g. a board to be tested. Preferably, said predetermined number of board samples are cut into pieces of a predetermined size. Preferably, said samples are converted to a cellulose-based board sample. Preferably, the obtained cellulose-based board sample is then analyzed, e.g. in a chamber.

Preferably, an amount ranging from 1 to 10 g, most preferably 2 to 3 g based on the amount of dry pulp or formed cellulose-based board is withdrawn from the production process, preferably from the formed cellulose-based board.

According to one embodiment, the invention encompasses a method for detecting and enumerating the number of bacteria, e.g. in the form of colonies based on digital camera technology and image processing is provided.

According to one aspect of the invention, at least one intermediate image of the cellulose-based board sample is captured, when a predetermined time period has elapsed since the first image was captured. Thanks to this aspect, it is easier and more reliable to detect bacterial growth and distinguish it from fibres, pigments etc., by comparing images over time.

According to one embodiment, comparison of images may comprise detecting bacterial growth on the final image by detecting colour changes or changes in other characteristic properties present on the final image but not on the firstly captured image. The detection of bacterial growth on the final image may correspond to a difference in number of detected bacterial colonies. According to one embodiment, comparison of the images with respect to the size of bacterial colonies is used to evaluate the bacterial growth wherein the size may correspond to the number of bacterial growth spots in the pulp sample, e.g. on the cellulose-based board sample.

According to one embodiment, the pulp sample, e.g. cellulose-based board sample is placed on a supporting structure in a chamber together with at least one other cellulose-based board sample, and wherein the supporting structure and/or image capturing means is movable whereby the respective sample is positioned to enable capturing of images of the respective samples, e.g. cellulose-based board samples.

### Brief description of the drawings

Fig. 1 shows a system for detection of bacterial growth in cellulose-based board samples.
Fig. 2 shows a movable supporting structure 3, configured for holding six cellulose-based board.
Fig. 3 shows a schematic set up of equipment for performing a method for detection of bacterial growth in cellulose-based board.
Fig. 4 illustrates an example of a method according to an embodiment.
Fig.5 shows a table presenting results from the detection.

### Detailed description of the preferred embodiments

Whereas the below detailed description essentially refers to cellulose-based board, it is to be understood a skilled person could apply the teachings herein to perform corresponding determination of bacterial growth with respect to any pulp sample withdrawn from the board manufacturing process. Preferably, a number of samples are taken out from the pulp, e.g. from a board to be tested. Samples may be taken out from the board according to standard methods at the mill following ISO standards, e.g. ISO 186:2002. A sample withdrawn every day during production of the board may be enough for some board qualities which also may be in line with requirements of some customers while other customers may require several samples to be withdrawn for tests.

The board samples are preferably cut into pieces of a predetermined size in accordance with known standard methods.

For example, a predetermined sample size could have a width of about 1 cm and a length of about 7-8 cm. The size of the sample piece is however not crucial.

Identical references are preferably added to each of the pieces. The pieces are weighed and the weight of each piece is thoroughly noted down.

Each piece of the board sample is converted to a cellulose-based board sample.

There are different methods for preparing the cellulose-based board samples from the board sample pieces. One example of an appropriate method is ISO 8784. However, modified versions of ISO 8784 or other ISO-standard methods may be used. In short, most of the different available methods comprise the steps of disintegration of each piece of the board sample in an appropriate diluent solution of a certain known volume. One appropriate solution may be Ringer's solution comprising sodium chloride, potassium chloride, calcium chloride, sodium hydrogen carbonate and water in appropriate proportions. However, other possible isotonic solutions exist and the choice of diluent solution may depend on the ISO-standard method chosen. The disintegration preferably continues until a suspension free from fiber clumps is obtained. Next step following the disintegration step may preferably be plating of the fiber suspension onto a suitable suspension carrier, e.g. a Petri dish of an appropriate size.

A culture medium preferably comprising agar is added to the fiber suspension in the carrier, e.g. the Petri dish. Ready-to-use commercial culture media may be used or a culture medium may be prepared on site wherein said medium may then comprise e.g. at least one of tryptone, yeast extract, dextrose, glucose agar and water. ISO 11133 provides guidelines on preparation and production of culture media. Final pH of the culture medium may preferably be within a pH ranging from 6.5 to 7.5, more preferably from 6.8 to 7.2, i.e. 7.0 ± 0.2.

The cellulose-based board samples are now ready for incubation and simultaneous detection of bacterial growth in an incubation and detection chamber according to the invention. Preferably, all samples are simultaneously detected in one chamber. According to one embodiment, at least one sample may be stored at appropriate conditions (preferably at low temperature conditions in a refrigerator) as a fallback if further/additional tests need to be performed after having received the results from the detection of bacterial growth. With respect to samples withdrawn from other stages in the board manufacturing process, e.g. in the head box or from the stock solution, such samples may be worked-up in a suitable manner. As an example, corresponding plating may be performed prior to subsequent addition of a culture medium.

According to one embodiment, samples from different stages in the board manufacturing process are withdrawn at the same point in time. Said samples are subsequently analyzed according to the claimed method but subsequently mutually compared to evaluate to what extent bacterial levels vary in the process and to more precisely determine bacterial growth from the combined results from the samples withdrawn from the different stages.

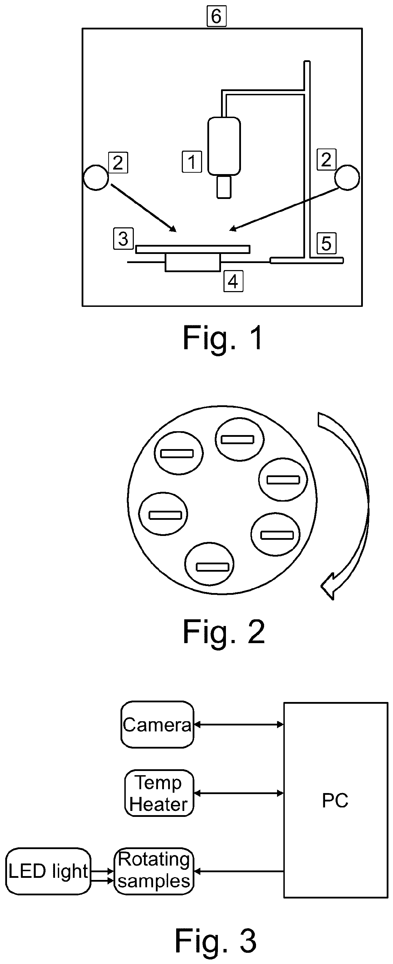

Fig. 1 is an illustration of a system for detection of bacterial growth in cellulose-based board sample/-s. The system comprises a camera 1, for taking a sequence of at least two images of a cellulose-based board sample. The camera 1 may be e.g. a video camera, a camera, a scanner, an infrared camera or similar device configured for capturing digital images.

The camera 1 may for example comprise e.g. a charge-coupled device (CCD)camera. The CCD camera is a device for movement of an electrical charge, usually from within the device to an area where the charge can be manipulated, for example conversion into a digital value.

Further, the system may comprise a source of illumination 2. The source of illumination 2 may comprise any arbitrary device, or set of devices, configured for emitting electromagnetic radiation within a certain portion of the electromagnetic spectrum, having a wavelength in the range about 390-700 nanometers, i.e. the visible light spectrum.

The source of illumination 2 may be e.g. a Light-Emitting Diode (LED), an incandescent light, a halogen light, a luminescent light, a neon light, a plasma light, a flash or a light tube.

According to one embodiment, the source of illumination 2 is activated only at the moment when an image is to be captured by the camera 1. Thereby energy is saved. Also, the cellulose-based board sample is not heated by light enabling more constant temperature during the process.

An advantage with LEDs as source of illumination 2 is that, in contrast to most other light sources, LEDs radiate very little heat in the form of IR that can transfer heat to the cellulose-based board sample. Wasted energy is dispersed as heat through the base of the LED. Further, LEDs have a relatively long useful life and emit more lumens per watt than e.g. incandescent light bulbs.

By using a set of illumination sources 2, i.e. a multitude of sources of illumination 2, and spreading them out in a distributed manner, an even and uniform illumination is achieved of the cellulose-based board sample, eliminating or at least reducing impact of shadows.

The source of illumination 2, or any additional source of illumination, may be situated under each, or at least some, cellulose-based board sample in some embodiments. By illuminating the sample from the underside, it may become easier to detect bacterial growth in the samples.

The cellulose-based board sample(s) may be placed on a supporting structure 3. The supporting structure 3 may be configured for holding at least one cellulose-based board sample. In some embodiments, the supporting structure 3 may be movable and configured to sequentially move a plurality of cellulose-based board samples in position for enable capturing of an image of the cellulose-based board sample by the camera 1.

In some embodiments, the supporting structure 3 may be approximately circular and cellulose-based board sample may be put into a correct position to enable capturing of images by camera 1, in some embodiments as will be further explained in Figure 2. However, in other embodiments, the supporting structure 3 may be rectangular, quadratic, oval, polygon, triangle, pentagon, ellipse, conic section, etc. Further, the cellulose-based board sample may be put in position for image capturing by the camera 1 by another moment than rotating the supporting structure 3, e.g. by a lateral movement. In embodiments wherein the supporting structure 3 is movable, such as e.g. rotatable, the movement may be controlled by a movement enabling device 4, such as e.g. an electric motor, which in turn may be controlled by a control unit, as will be further described in Figure 3.

The camera 1 may be kept stabilized and fixed, in some embodiments, by a fixing device 5, such as e.g. a stand, a tripod, a tubing construction or similar.

The cellulose-based board sample(s) is kept in a chamber 6. The chamber 6 may comprise a closed volume of air within which at least one environmental parameter such as temperature, humidity, air pressure, oxygen level, etc. is kept constant. As an example, the chamber 6 may comprise a cabinet, a fume hood, etc. The chamber 6 may be at least partially transparent Plexiglas, glass, transparent plastic, etc.

An advantage with keeping the cellulose-based board sample in the chamber 6, and keeping the chamber 6 encapsulated, is that contamination of the cellulose-based board sample is eliminated, or at least reduced.

The camera 1 may be situated inside the chamber 6 in some embodiments, or alternatively outside of the chamber 6. The chamber 6 is preferably transparent or has a transparent part where the camera 1 is mounted on a fixing device 5, arranged for capturing images of the cellulose-based board sample in the chamber 6. The system in Figure 1 may thus be used for performing a method for detection of bacterial growth in cellulose-based board. When a cellulose-based board is produced, a cellulose-based board sample is extracted from the cellulose-based board as described above. An identity reference may be added to the cellulose-based board sample in order to associate it with the cellulose-based board from which it has been extracted.

The cellulose-based board sample is then placed in the chamber 6, e.g. on the supporting structure 3. In some embodiments, a multitude of samples from the same or different cellulose-based boards may be extracted and put on the supporting structure 3. The environment in the chamber 6 may be kept at predetermined values, such as at a predetermined temperature. The predetermined temperature may be set to a temperature within a range from 20 ºC to 42 ºC, preferably within a range from 32 ºC to 37 ºC. The camera 1 may capture a first image or picture of the cellulose-based board sample. The captured first image may be stored in a computer memory or database, associated with the identity of the cellulose-based board sample, optionally together with a time stamp or similar.Another subsequently captured image, preferably a final image, may then be taken by the camera 1 after a predetermined time period, such as e.g. after about 72 hours, 48 hours, 24 hours or somewhere in between. Also this final image may be stored in the computer memory or database, associated with the identity of the cellulose-based board sample, possibly together with a time stamp or similar. The two images of the cellulose-based board sample may then be compared by an image recognition program, i.e. a computer program configured for computer vision and object recognition, for detecting bacterial growth on the cellulose-based board sample. The detected bacterial growth may be a number describing a number of detected bacterial colonies on the final image of the cellulose-based board sample. Thus, if the number is zero, no bacterial growth has occurred, or at least has not been found. If this result seems unreasonable, another test may be performed by making use of at least one sample stored at appropriate conditions, as earlier described.

Computer vision is a technical field comprising methods for acquiring, processing, analysing, and understanding images and, in general, high-dimensional data from the real world in order to produce numerical or symbolic information. A theme in the development of this field has been to duplicate the abilities of human vision by electronically perceiving and understanding an image. Understanding in this context means the transformation of visual images (the input of retina) into descriptions of the world that can interface with other thought processes and elicit appropriate action.

This image understanding can be seen as the disentangling of symbolic information from image data using models constructed with the aid of geometry, physics, statistics, and learning theory. Computer vision may also be described as the enterprise of automating and integrating a wide range of processes and representations for vision perception. The image data of the camera 1 may take many forms, such as e.g. images, video sequences, views from multiple cameras, or multidimensional data from a scanner.

According to one embodiment, image recognition may comprise detecting bacterial growth on the final image by detecting colour changes or changes in other characteristic properties present on the final image but not on the firstly captured image. The detection of bacterial growth on the final image may be a number of detected bacterial colonies on the final image of the sample. Thus the size of the bacterial growth may comprise the number of bacterial growth spots on the cellulose-based board sample.

Thereby, based on the detected bacterial growth on the cellulose-based board sample, a rate of bacterial growth may be determined based on the predetermined time period such as e.g. after about 72 hours, 48 hours, 24 hours or somewhere in between, when the final image has been captured after the firstly captured image.

This determined rate of bacterial growth may be compared with a pre-determined limit value and when the pre-determined limit value is exceeded, the board associated with the cellulose-based board sample may be determined not to be delivered to the customer. As a non-limiting example, two bacterial colonies may be detected on the final image of the cellulose-based board sample.

This number, i.e. two, may be compared with the pre-determined limit value, which may be one. As the pre- determined limit value thus is exceeded, the board associated with the cellulose-based board sample is not delivered to the customer.

Fig. 2 illustrates a movable supporting structure 3, configured for holding six cellulose-based boards. A control unit may send control signals to the movement enabling device 4, i.e. an electric motor, for sequentially turning the movable supporting structure 3 in order to bring the respective cellulose-based board sample in position for enable image capturing by the camera 1.

The illustrated embodiment is merely an arbitrary example. Another number *n* of cellulose-based board samples 1 < *n* < ∞ may be held by the supporting structure 3. The supporting structure 3 may have any arbitrary form besides circular in different embodiments. Fig. 3 illustrates a schematic set up of equipment for performing a method for detection of bacterial growth in cellulose-based board. A control unit such as e.g. a computer or similar may control and/ or communicate with various other entities over a wired or wireless connection. Such devices may be e.g. the camera 1, a thermometer for measuring the temperature within the chamber 6, a temperature adjusting device for heating/ cooling the temperature in the chamber 6 in order to keep the temperature constant and at the predetermined temperature when the method is performed, based on input from the thermometer. Further, the control unit may send control signals for moving the supporting structure 3, via the movement enabling device 4, for putting cellulose-based board samples situated on the supporting structure 3 in correct position for enabling image capturing, in some embodiments.

The control unit may in some embodiments control a light source in order to keep a constant light of the cellulose-based board samples. Alternatively, the light source may be activated constantly, in some embodiments.

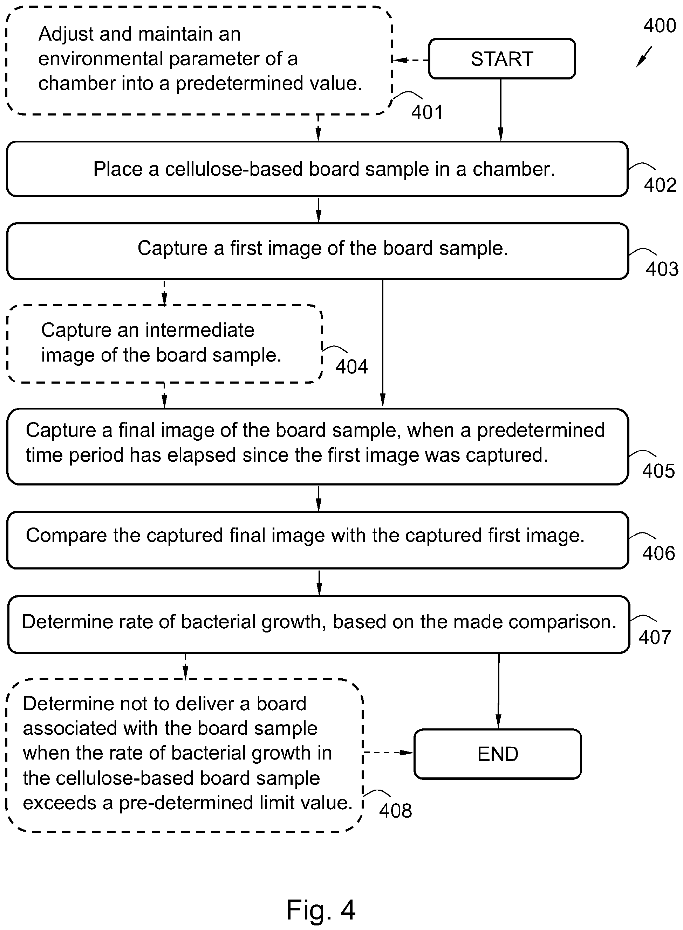

Figure 4 illustrates an example of a method 400 according to an embodiment. The flow chart in Figure 4 shows a method 400 for detection of bacterial growth in cellulose-based board.

The method 400 may be at least partly performed in, or supervised by, a control unit, e.g. in a computer.

The cellulose-based board may be any arbitrary kind of cellulose-based product. According to one embodiment, to enable detection of bacterial growth in cellulose-based board, the method 400 may comprise a number of steps 401-408. However, some of these steps 401-408 may be performed solely in some alternative embodiments, like e.g. step 401, 404 and/ or 408. Further, the described steps 401-408 may be performed in a somewhat different chronological order than the numbering suggests. The method 400 may comprise the subsequent steps:
Step 401, which may be performed only in some embodiments, comprises adjusting at least one environmental parameter of the chamber 6 into a predetermined value, and maintaining the environmental parameter at the predetermined value.

The environmental parameter may be e.g. temperature, humidity, pH, air pressure, oxygen level, etc.

Step 402 comprises placing a cellulose-based board sample in a chamber 6.

The cellulose-based board sample may preferably be marked with an identification code, enabling an association with a particular board from which the cellulose-based board sample has been extracted. The identification code may be a non-repeatable code applied e.g. on the back side of the cellulose-based board sample, such as a number, a colour code, etc.

Thereby, the board affected by bacterial growth may be identified, based on detected bacterial growth in the cellulose-based board sample.

The cellulose-based board sample may be placed on a supporting structure 3 in the chamber 6 together with at least one other cellulose-based board sample.

The supporting structure 3 may be movable for placing the respective cellulose-based board sample in position to enable capturing of images of the respective cellulose-based board samples.

Step 403 comprises capturing a first image of the cellulose-based board sample with a camera 1. The camera 1 may be e.g. a video camera, a camera like e.g. a digital camera, etc. The image may be captured as a digital image and stored in a memory and/ or database, associated with an identity reference of the cellulose-based board sample and possibly a time code or similar, in some embodiments.

Step 404, which may be performed according to one embodiments, comprises capturing at least one intermediate image of the cellulose-based board sample, when a predetermined time period has elapsed since the first image was captured 403, in some embodiments.

The predetermined time period for capturing at least one intermediate image of the cellulose-based board sample may be about e.g. 30 minutes, 1 hour, 2 hours, 8 hours, 12 hours, 24 hours, or any arbitrary time period of any length in between these values. This also means that an image may be captured every half hour, every hour, every second hour or every 8^{th} hour etc. until a final image has been captured. It is further understood that the predetermined time may be shorter than 30 minutes and may for example be every 10 minutes.

Step 405 comprises capturing the final image of the cellulose-based board sample, when a predetermined total time period has elapsed since the first image was captured 403.

The predetermined total time period may be e.g. 72 hours, 48 hours, 24 hours or somewhere in between in different embodiments. Step 406 comprises comparing the captured 405 final image with the captured 403 first image.

According to one embodiment, captured 405 final image is compared with the captured 403 first image. By means of the comparison, various miscolouring etc., of the cellulose-based board sample may be filtered away so as to obtain an improved analysis. Step 407 comprises determining a rate of bacterial growth, based on the made comparison 406.

The comparison 406 and the determination of the rate of bacterial growth may be made by an image recognition program running in the control unit. Further, the rate of bacterial growth may be determined by detecting bacteria on the captured 405 final image, and estimating the amount of detected bacteria on the captured 405 final image.

The bacterial growth may thus comprise the number of bacterial colonies detected on the captured 405 final image of the sample, as the final image is captured 405 at a (total) predetermined time period after the captured 403 first image.

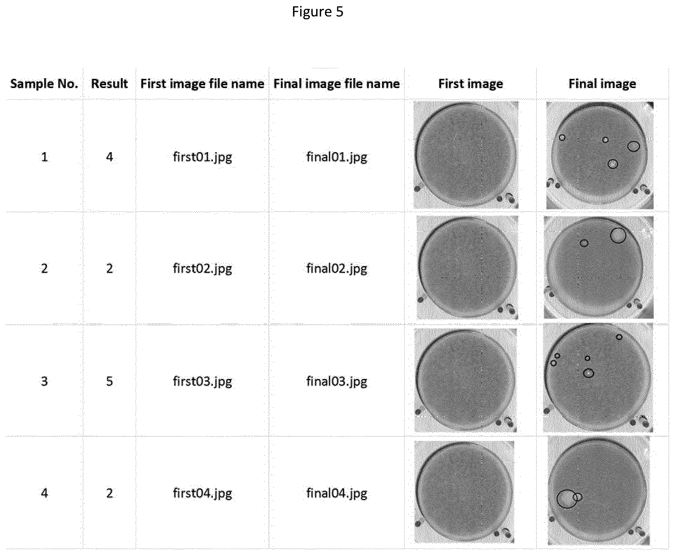

Step 408, which may be performed only in some embodiments, comprises determining not to deliver a board associated with the cellulose-based board sample when the determined 407 rate of bacterial growth in the cellulose-based board sample exceeds a pre-determined limit value. The result from the detection of each sample may e.g. be presented as a number of colonies in each sample, or as a bacterial growth rate by dividing the number of colonies may and the predetermined time period, or as number of colonies per weight dry sample, and the results may be reported to the customer or only used in-house as a quality check to determine whether the board fulfills the set quality standards or not. A detection of bacterial growth in cellulose-based board according to the method and by using the control unit and system was performed. Four samples were prepared from the board samples withdrawn from the production line at the mill and in accordance with the ISO standards mentioned above. In Fig.5 a table is shown presenting results from the detection.

The table comprises six columns termed Sample No., Result, First image file name, Final image file name, First image, Final image. The four board samples, samples No. 1-4, were detected according to the inventive method. For each sample a first and a final image were taken. All images were captured and saved in a jpg-format and the images where given names corresponding to the sample numbers also including information on the captured image being a first or a final image. For instance, the file name "first01.jpg" is to be understood as the first image of sample No. 1, whereas the file name "final04.jpg" should be understood as the final image of sample No. 4. In the Result-column the result from the detection is presented as a number of colonies in each sample and the number is hence an integer. For sample No. 1 the integer "4" is presented in the column Result, and in the last column, the column for Final image, four colonies are visible in the image having the file name final01.jpg. Since the first image, first01.jpg, shows no colonies this means that four colonies have been detected by the method in sample No. 1. In sample No. 2, two colonies have been detected and in the column Result presented as "2", in sample No. 3 the result was five colonies and in sample No. 4, two colonies have been detected. As can be seen in the column First image, no colonies are seen in the first images. In the final images the colonies are visible as white, almost circular spots. Since some colonies are clearly visible in the images in the table while other, smaller colonies are more difficult to see, the colonies in the images shown in the table have been marked-up by drawing a circle around every colony. However, on the real images analyzed for instance on a computer screen it is much easier to see and to count the colonies in the images.

The table in Fig. 5 is one of many ways to present the result from the detection and is in no way limiting the scope of the invention but instead to be seen as an example. It is further to be understood that the result is to be compared with a pre-determined limit value. If, for instance, the pre-determined limit value is set to a maximum of three colonies in the final image, this then would mean that sample Nos. 1 (four colonies) and 3 (five colonies) exceed the pre-determined limit value and the board associated with said two samples should not be delivered to the customer, or at least be detected again by preparing new samples originating from the board in question. If the new result again shows that the number of colonies detected is too high, i.e. exceeding the pre-determined limit value, the board will indeed not be delivered to the customer.

As will be understood by those skilled in the present field of art, numerous changes and modifications may be made to the above described and other embodiments of the present invention, without departing from its scope as defined in the appending claims. For example, in some embodiments it may be preferred to over time change the temperature within the chamber and preferably at a certain temperature change velocity from a higher temperature to a lower temperature or vice versa, e.g. from 37 ºC down to 32 ºC or from 32ºC up to 37 ºC during the predetermined time period for detecting the bacterial growth. Use of an additional camera lens may be needed to increase the magnification, e.g. up to 10 times, to facilitate the image quality of the images captured. It is also to be understood that different file formats may be used and not only the jpg-format as presented in relation to Figure 5. Other formats may be tif, png, gif, bmp, raw etc. Various aspects and embodiments of the present invention are defined by the following numbered clauses.

## Claims

1. A method (400) for detection of bacterial growth in a sample of i) pulp or ii) cellulose-based board comprising
i) optionally placing (402) said sample in a chamber (6);
ii) capturing (403) a first image of said sample;
iii) capturing (405) a final image of said sample when a predetermined total time period has elapsed since the first image was captured (403);
iv) comparing (406) the captured (405) final image with the captured (403) first image; and
v) determining (407) a rate of bacterial growth, based on the comparison (406) of step iv).

2. The method (400) according to claim 1, wherein the sample of pulp is a sample of i) a cellulose-based board, ii) a head box solution, or iii) a stock solution.

3. The method (400) according to any of claim 1 or 2, further comprising:
adjusting (401) at least one environmental parameter of the chamber (6) into a predetermined value, and maintaining the environmental parameter at the predetermined value.

4. The method (400) according to any of claims 1-3, further comprising:
capturing (404) at least one intermediate image of said sample, when a predetermined time period has elapsed since the first image was captured (403).

5. The method (400) according to any of claims 1-4, wherein a cellulose-based board is placed (402) on a supporting structure (3) in the chamber (6) together with at least one other sample of a cellulose-based board; and wherein the supporting structure (3) is movable for placing the respective sample of a cellulose-based board in position to enable capturing (403, 404, 405) of images of the respective sample of a cellulose-based board.

6. A control unit for detection of bacterial growth in a sample of i) pulp or ii) a cellulose-based board, which control unit is configured to:
i) generate a control signal to capture a first image of the sample of a) pulp or b) cellulose-based board;
ii) generate a control signal to capture a final image of said sample, when a predetermined time period has elapsed since the first image was captured;
iii) compare the captured final image with the captured first image;
and;
iv) determine a rate of bacterial growth, based on the made comparison.

7. The control unit according to claim 6 further configured to adjust at least one environmental parameter of a chamber (6) into a predetermined value, and maintain the environmental parameter at the predetermined value.

8. The control unit according to claim 6 or 7, further configured to generate a control signal to capture at least one intermediate image of said sample, when a predetermined time period has elapsed since the first image was captured.

9. The control unit according to any of claims 6-8, further configured to generate control signals to move a supporting structure (3) in the chamber (6), in order to position said sample of a cellulose-based board to enable capturing (403, 404, 405) of images of the respective sample.

10. A system for detection of bacterial growth in a sample of a) pulp or b) a cellulose-based board, comprising:
i) optionally a chamber (6) for accommodating said sample;
ii) a camera (1) configured to capture images of said sample comprising at least one bacterium; and a control unit according to any of claims 6-9.

11. The system according to claim 10, further comprising:
a measuring instrument for measuring at least one environmental parameter of the chamber (6); and an environmental adjustment device for adjusting at least one environmental parameter of the chamber (6) in order to maintaining the environmental parameter at a constant value.

12. The system according to any of claim 10 or 11, further comprising:
a supporting structure (3) for holding said sample when placed in the chamber (6), wherein the supporting structure (3) is movable.
